# EUROPÄISCHE PATENTSCHRIFT

(11) **EP 1 853 182 B1**
(45) Veröffentlichungstag und Bekanntmachung des Hinweises auf die Patenterteilung: **10.04.2013**
(21) Anmeldenummer: 06706912.0
(22) Anmeldetag: 14.02.2006
(51) Int. Cl.: A61B 17/74, A61B 17/80

(54) **ORTHOPÄDISCHES FIXIERSYSTEM**
ORTHOPEDIC FIXATION SYSTEM
SYSTEME DE FIXATION ORTHOPEDIQUE

(30) Priorität: 19.02.2005 DE 102005007674
(43) Veröffentlichungstag der Anmeldung: 14.11.2007
(73) Patentinhaber: Aesculap AG, 78532 Tuttlingen (DE)
(72) Erfinder: HOEGERLE, Roland, Alois, 78532 Tuttlingen (DE); ALTERMANN, Frank, 78532 Tuttlingen (DE); STEDTFELD, Hans-Werner, 90475 Nürnberg (DE); PARKER, Martyn, Peterborough PE6 7NJ (GB)
(74) Vertreter: Hoeger, Stellrecht & Partner Patentanwälte
(86) Internationale Anmeldenummer: PCT/EP2006/001304
(87) Internationale Veröffentlichungsnummer: WO 2006/087159

(56) Entgegenhaltungen:
- WO-A-2004/075766
- WO-A-2004/093700
- US-A- 4 438 762
- US-A1- 2001 049 528
- US-A1- 2003 171 754
- US-B1- 6 712 818
- PATENT ABSTRACTS OF JAPAN Bd. 2003, Nr. 12, 5. Dezember 2003 (2003-12-05) & JP 2004 097446 A (FOR S MEDICAL:KK), 2. April 2004 (2004-04-02)

## Beschreibung

Die Erfindung betrifft ein orthopädisches Fixiersystem zum Festlegen eines ersten Knochenfragmentes an einem zweiten Knochenfragment mit einer in das erste Knochenfragment mittels eines einen Gewindegang aufweisenden Knochengewindes einschraubbaren Tragschraube, mit einer die Tragschraube umgebenden und diese in Längsrichtung frei verschieblich führenden Traghülse und mit einer mindestens eine Gewindebohrung aufweisenden, am zweiten Knochenfragment festlegbaren Knochenplatte, in deren Gewindebohrung die Traghülse mittels eines Außengewindes einschraubbar ist.

Zum Fixieren von zwei Knochenfragmenten relativ zueinander, beispielsweise zwei Knochenfragmenten im Bereich des Schenkelhalses eines Femurknochens, ist es bekannt, in eines der beiden Knochenfragmente beispielsweise den Gelenkkopf des Femurs, eine Tragschraube einzuschrauben und diese in einer Traghülse längsverschieblich zu führen, die am zweiten Knochenfragment festgelegt ist, also beispielsweise am oben Teil des Femurs, so daß die Tragschraube in der Traghülse geführt wird, aber eine Längsverschiebung zuläßt.

Bei bekannten orthopädischen Fixiersystemen dieser Art ist die Traghülse einstückig mit einer Knochenplatte verbunden, welche am zweiten Knochenfragment festgelegt werden kann (US 2,702,543, US 2,612,159). Es gibt auch Fixiersysteme, bei denen die Traghülse in die Knochenplatte eingeschoben werden kann (US 4,438,762) und bei anderen Systemen wird die Traghülse unmittelbar in den Knochen eingeschraubt (US 4,940,467).

Eine andere Möglichkeit der Festlegung der Traghülse an der Knochenplatte kann vorsehen, daß die Traghülse in die Knochenplatte eingeschraubt ist. Da die Knochenplatte nur eine geringe Dicke hat, müssen im Bereich der Schraubverbindung alle Biegekräfte von der Traghülse auf die Knochenplatte übertragen werden, es ist also notwendig, in diesem Bereich ein stabiles Maschinengewinde anzuordnen.

Andererseits wird die Tragschraube üblicherweise mit einem sogenannten Knochengewinde in den Knochen eingeschraubt, es handelt sich dabei meistens um ein selbstschneidendes Gewinde mit relativ hoher Steigung und relativ großem Abstand benachbarter Gewindegänge. Es sind daher getrennte Arbeitsgänge notwendig zum Einschrauben der Tragschraube in das erste Knochenfragment und zum Einschrauben der Traghülse in die Knochenplatte (WO2004/075766 A1, Basis für dem Oberbegriff des Anspruchs 1).

Es ist Aufgabe der Erfindung, ein orthopädisches Fixiersystem der gattungsgemäßen Art so zu verbessern, daß das Implantieren dieses Systems erleichtert wird.

Diese Aufgabe wird bei einem orthopädischen Fixiersystem der eingangs beschriebenen Art erfindungsgemäß dadurch gelöst, dass das Knochengewinde einerseits und die Gewindebohrung und das Außengewinde andererseits dieselbe Steigung der Gewindegänge aufweisen und dass die Gewindebohrung sowie das Außengewinde mindestens zwei nebeneinanderliegende Gewindegänge mit entsprechend geringerer Breite jedes Gewindeganges aufweisen .

Durch die Verwendung von Gewinden mit derselben Steigung können Tragschraube und Traghülse gemeinsam in das erste Knochenfragment bzw. die Knochenplatte eingeschraubt werden, ohne daß sich dabei ihre Relativposition in axialer Richtung verändert. Andererseits erfährt die Traghülse in der Innengewindebohrung der Knochenplatte eine besonders gute Fixierung dadurch, daß nicht nur ein Gewindegang vorgesehen ist, sondern nebeneinander mindestens zwei Gewindegänge, vorzugsweise drei Gewindegänge. Die Erhöhung der Zahl der Gewindegänge hat außerdem den Vorteil, daß es längs des Umfanges der Traghülse mehrere Anfangspunkte für Gewindegänge gibt, so daß das Außengewinde in der Innengewindebohrung bereits nach einer Umdrehung greifen kann, deren Winkel deutlich geringer ist als 360°, maximal entspricht dieser Winkel dem Quotienten aus einer vollen Umdrehung und der Zahl der Gewindegänge.

Die Knochenplatte kann mehrere Gewindebohrungen zur Aufnahme von Traghülsen aufweisen, so daß dem Operateur nach Anlegen der Knochenplatte an das zweite Knochenfragment mehrere Möglichkeiten gegeben sind, Traghülsen und damit Tragschrauben zu implantieren, gegebenenfalls können mehrere Traghülsen mit Tragschrauben nebeneinander implantiert werden.

Es ist günstig, wenn die Traghülse eine kreiszylindrische Innenwand aufweist, die sich eng an die kreiszylindrische Außenwand eines Schaftabschnittes der Tragschraube anlegt. Dadurch ergibt sich eine allseitige Führung der Tragschraube in der Traghülse.

Besonders vorteilhaft ist es, wenn die kreiszylindrische Innenwand und der kreiszylindrische Schaftabschnitt sich zum Außengewinde der Traghülse hin stufenförmig erweitern, so daß die entstehende Stufe einen Anschlag ausbildet, der die Ausziehbewegung der Tragschraube aus der Traghülse in Richtung auf das Knochengewinde begrenzt.

Die Tragschraube weist vorzugsweise an ihrem dem Knochengewinde gegenüberliegenden Ende eine Aufnahmeöffnung zum formschlüssigen Einführen eines Werkzeuges auf, es kann sich dabei beispielsweise um einen Innensechskant handeln. Weiterhin ist es günstig, wenn die Traghülse an ihrem Außengewinde eine Erweiterung in ihrer Innenwand aufweist zum formschlüssigen Einführen eines Drehwerkzeuges. Auch diese Erweiterung kann als Innensechskant in der Traghülse ausgebildet sein.

Die nachfolgende Beschreibung bevorzugter Ausführungsformen der Erfindung dient im Zusammenhang mit der Zeichnung der näheren Erläuterung. Es zeigen:

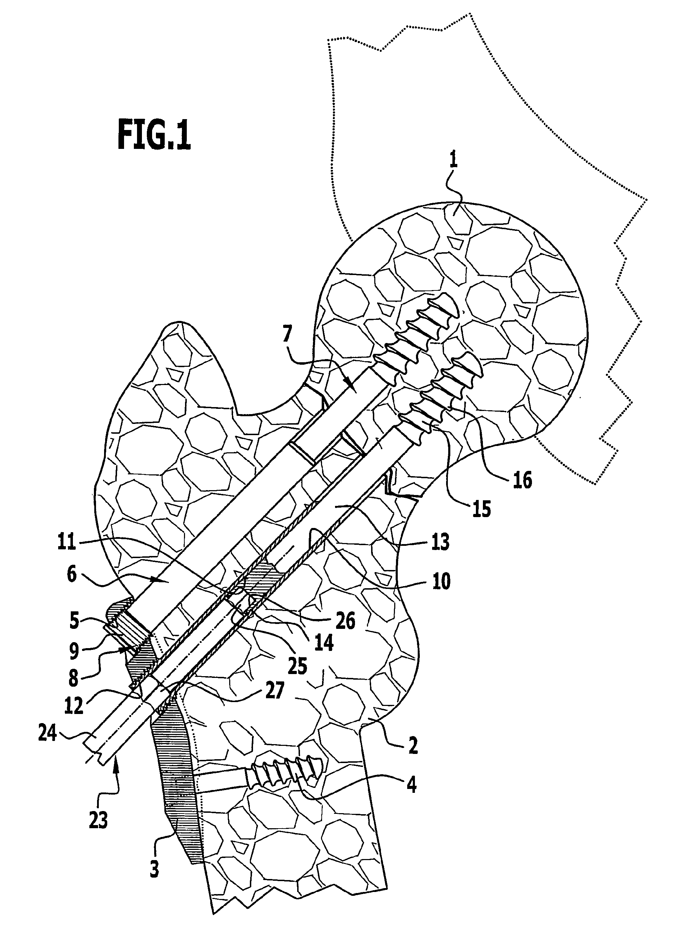

- Figur 1:: eine Querschnittsdarstellung durch einen Femurknochen im Bereich des Schenkelhalses mit einer am Femur festgelegten Knochenplatte und zwei in diese eingeschraubten Traghülsen mit je einer Tragschraube;
- Figur 2:: eine perspektivische Ansicht der Knochenplatte der Figur 1 mit einer in Längsrichtung aufgeschnittenen Traghülse vor dem Einschrauben in die Knochenplatte und mit einer Tragschraube in der Traghülse und
- Figur 3:: vergrößerte Darstellungen des Knochengewindes der Tragschraube und des Außengewindes der Traghülse.

Das orthopädische Fixiersystem wird am Beispiel eines Schenkelhalsbruches am Femur erörtert, bei einem solchen Schenkelhalsbruch ist es notwendig, den die Gelenkkugel tragenden Teil des Femurs, also ein erstes Knochenfragment 1, relativ zum übrigen Femur, also einem zweiten Knochenfragment 2, festzulegen und dabei zwischen den beiden Knochenfragmenten eine gewisse Bewegung in der Verbindungsrichtung der beiden Knochenfragmente zu ermöglichen, da diese Bewegung die Ausbildung von Knochensubstanz anregt. Die Verbindung darf daher keine starre Verbindung sein, sondern soll eine Verbindung sein, durch die die Knochenfragmente zwar relativ zueinander geführt sind, in ihrer Verbindungsrichtung aber geringe Bewegungen ausführen können.

Das orthopädische Fixiersystem umfaßt eine Knochenplatte 3, die an der Außenseite des zweiten Knochenfragmentes 2 auf der dem ersten Knochenfragment 1 gegenüberliegenden Seite festgelegt wird. Die Knochenplatte 3 kann an die Außenkontur des zweiten Knochenfragmentes 2 angepaßt sein und wird dort in herkömmlicher Weise mittels einer oder mehrerer Knochenschrauben 4 festgelegt. Die Knochenplatte 3 weist in dem dargestellten Ausführungsbeispiel in einem oberen Teil vier nebeneinanderliegende Innengewindebohrungen 5 auf, die gegenüber der Anlagefläche der Knochenplatte 3 alle in gleicher Weise geneigt sind, die Längsachsen der Innengewindebohrung 5 sind beispielsweise um 130° gegenüber der Anlagefläche der Knochenplatte 3 geneigt und verlaufen parallel zueinander.

Das orthopädische Fixiersystem umfaßt weiterhin mindestens eine längliche Traghülse 6 und jeweils jeder Traghülse 6 zugeordnet eine Tragschraube 7. Die Traghülsen 6 und die Tragschrauben 7 sind bei Verwendung von mehreren Traghülsen und Tragschrauben gleich ausgebildet, es wird daher nachstehend nur ein Exemplar ausführlich erörtert.

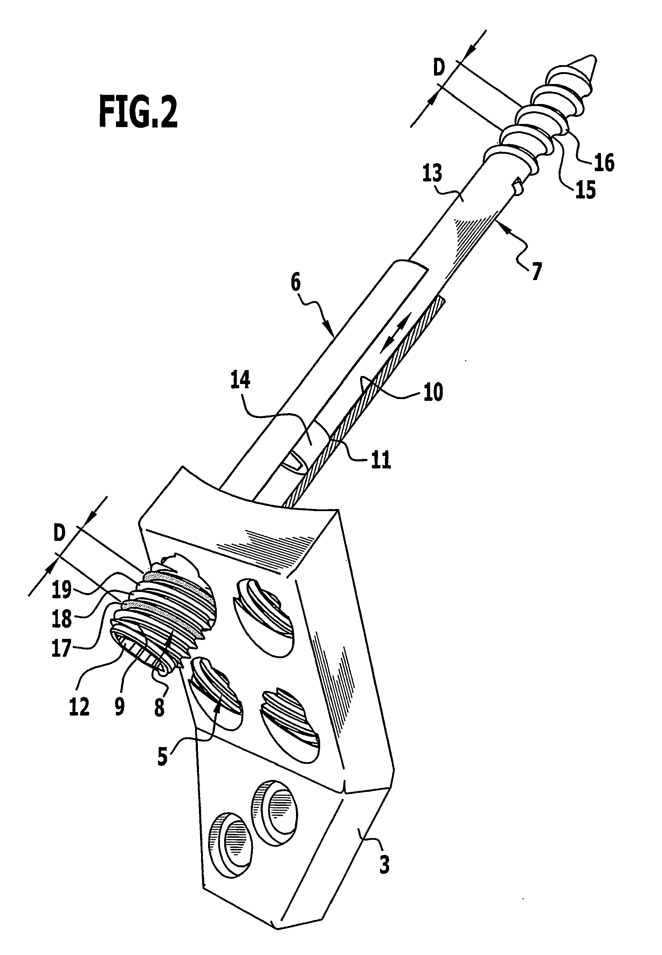

Die Traghülse 6 weist einen nach außen hin verdickten Einschraubbereich 8 an einem Ende auf, in diesem Einschraubbereich 8 trägt sie ein Außengewinde 9, mit dem die Traghülse 6 jeweils in eine Innengewindebohrung 5 einschraubbar ist. Die Innenwand 10 der Traghülse 6 ist kreiszylindrisch ausgebildet und erweitert sich etwa in der Mitte der Traghülse 6 in Richtung auf den Einschraubbereich 8 stufenförmig, so daß sich am Übergang der kreiszylindrischen Abschnitte mit unterschiedlichem Durchmesser eine Ringstufe 11 mit geringer Höhe ergibt (Figur 2).

Im Einschraubbereich 8 erweitert sich die Innenwand 10 noch einmal und bildet dort eine Erweiterung 12 mit einem Innensechskantquerschnitt aus, diese Erweiterung 12 schließt sich unmittelbar an das außengewindeseitige Ende der Traghülse 6 an.

Im Innern der Traghülse 6 ist die Tragschraube 7 aufgenommen, diese weist einen kreiszylindrischen Schaft 13 mit einem einen geringfügig größeren Durchmesser aufweisenden kreiszylindrischen Kopf 14 auf sowie an dem dem Kopf 14 gegenüberliegenden Ende ein vorzugsweise selbstschneidendes Knochengewinde 15. Der Außendurchmesser des kreiszylindrischen Schaftes 13 entspricht dem Innendurchmesser der Innenwand 10 im engeren, dem Einschraubbereich 8 gegenüberliegenden Teil der Traghülse 6, der Außendurchmesser des kreiszylindrischen Kopfes 14 entspricht dem Durchmesser der Innenwand 10 im Bereich zwischen der Ringstufe 11 und der Erweiterung 12, so daß die Tragschraube 7 in der Traghülse 6 in Längsrichtung frei verschieblich geführt ist, wobei ein Ausziehen der Tragschraube 7 aus der Traghülse 6 in beiden Richtungen verhindert ist, in der Richtung auf das Knochengewinde 15 hin durch ein Anschlagen des Kopfes 14 an der Ringstufe 11, in umgekehrter Richtung durch das Anschlagen des Knochengewindes 15 an der Traghülse 6.

Das Knochengewinde 15 weist einen Gewindegang 16 mit relativ hoher Steigung auf, beispielsweise mit einer Steigung von 3 mm pro voller Umdrehung.

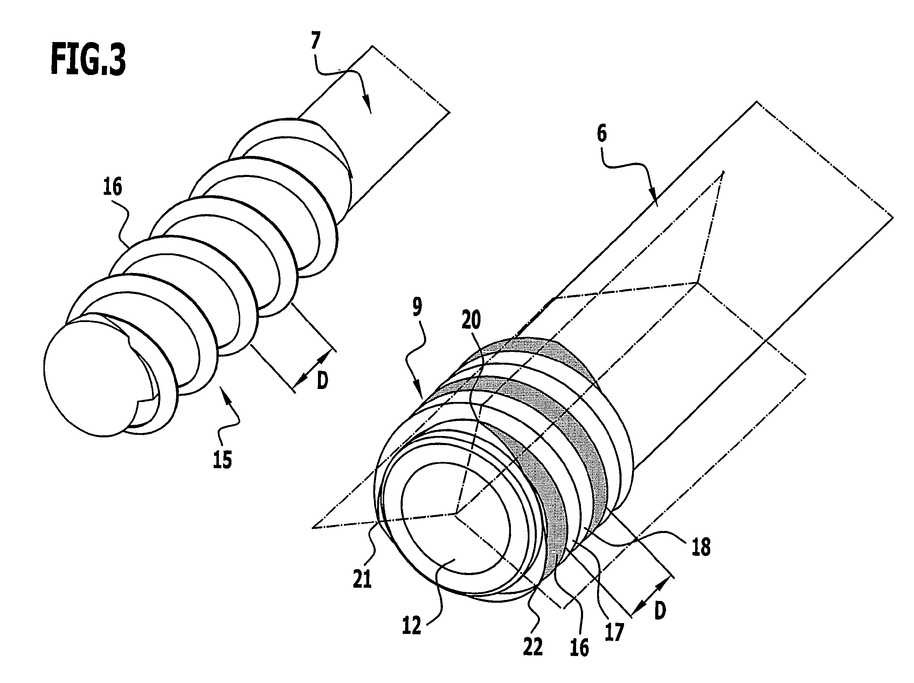

Auch das Außengewinde 9 weist dieselbe hohe Steigung auf, jedoch umfaßt das Außengewinde 9 in dem in der Zeichnung dargestellten Ausführungsbeispiel drei nebeneinanderliegende Gewindegänge 17, 18, 19, von denen einer in den Figuren 2 und 3 zur Verdeutlichung dunkel dargestellt ist. Die drei Gewindegänge 17, 18, 19 weisen eine Breite auf, die nur einem Drittel der Breite des Gewindeganges 16 entspricht, außerdem sind die Anfänge 20, 21, 22 der Gewindegänge 17, 18, 19 jeweils um 120° in Umfangsrichtung der Traghülse 6 gegeneinander versetzt (Figur 3). Benachbarte Abschnitte desselben Gewindeganges haben also denselben Abstand D (Figur 3), und zwar sowohl bei dem Knochengewinde 15 als auch bei dem Außengewinde 9, der Abstand benachbarter Gewindegänge beträgt jedoch bei dem Außengewinde nur ein Drittel des Abstandes bei dem Knochengewinde. Durch die größere Anzahl von benachbarten Gewindegängen ist das Außengewinde 9 in das in gleicher Weise mit drei Gewindegängen versehene Innengewinde der Innengewindebohrung 5 so fest eingeschraubt, daß alle mechanischen Belastungen im Verbindungsbereich gut übertragen werden können.

Zum Ein- und Ausdrehen der Traghülse 6 und der Tragschraube 7 wird ein Drehinstrument 23 verwendet, von dem in der Zeichnung nur ein länglicher Schaft 24 dargestellt ist, der an seinem freien Ende einen Außensechskant 25 trägt. Dieser paßt formschlüssig in einen Innensechskant 26 im Kopf 14 der Tragschraube 7.

Außerdem trägt der Schaft 24 im Abstand zum Außensechskant 25 einen Außensechskant 27, der formschlüssig in die Erweiterung 12 paßt.

Zum Implantieren des orthopädischen Fixiersystems wird zunächst die Knochenplatte 3 an der Außenseite des zweiten Knochenfragmentes 2 mit Hilfe einer Knochenschraube 4 festgelegt. Danach wird - gegebenenfalls nach dem Einbringen einer entsprechenden Bohrung - die Baueinheit aus Traghülse 6 und Tragschraube 7 eingesetzt. Zu diesem Zweck wird das Drehinstrument 23 mit seinem Schaft 24 so in die Traghülse 6 eingeführt, daß die Außensechskante 25 und 27 in den Innensechskant 26 bzw. die Erweiterung 12 drehfest eingreifen, dies ist entsprechend der Dimensionierung dann zu erreichen, wenn die Tragschraube 7 vollständig aus der Traghülse 6 herausgeschoben ist, wenn also der Kopf 14 an der Ringstufe 11 anliegt. Die Traghülse 6 und die Tragschraube 7 werden von dem Drehinstrument 23 gemeinsam verdreht, während zunächst die Tragschraube 7 mit dem Knochengewinde 15 in das erste Knochenfragment 1 eingeschraubt wird. Am Ende dieses Einschraubvorganges gelangt auch das Außengewinde 9 in den Bereich der Innengewindebohrung 5, das Außengewinde 9 greift dabei in das Innengewinde der Innengewindebohrung 5 und beim weiteren Verdrehen wird dadurch die Traghülse 6 mit dem Außengewinde in die Innengewindebohrung eingeschraubt, und zwar mit derselben Steigung wie die Knochenschraube 4 in das erste Knochenfragment 1 eingeschraubt wird, die Traghülse 6 und die Tragschraube 7 werden also in axialer Richtung bei diesem Einschraubvorgang nicht gegeneinander verschoben.

Die Traghülse 6 kann an ihrem außengewindeseitigen Ende noch eine Erweiterung aufweisen, die die maximale Einschraubtiefe bestimmt. Nach dem Einschrauben wird das Drehinstrument 23 aus der Traghülse 6 herausgezogen und diese wird mittels eines in der Zeichnung nicht dargestellten Stopfens verschlossen. In dieser Einbaulage werden die beiden Knochenfragmente relativ zueinander festgelegt, es ist möglich, daß das erste Knochenfragment geringfügig in Richtung auf das zweite Knochenfragment verschoben wird, wenn der Knochen belastet wird, so daß durch diese geringfügige Verschiebung und die damit verbundene zusätzliche Belastung im Kontaktbereich der beiden Knochenfragmente das Knochenwachstum angeregt werden kann.

## Patentansprüche

1. Orthopädisches Fixiersystem zum Festlegen eines ersten Knochenfragmentes(1) an einem zweiten Knochenfragment (2) mit einer in das erste Knochenfragment (1) mittels eines einen Gewindegang (16) aufweisenden Knochengewindes (15) einschraubbaren Tragschraube (7), mit einer die Tragschraube (7) umgebenden und diese in Längsrichtung frei verschieblich führenden Traghülse (6) und mit einer mindestens eine Gewindebohrung (5) aufweisenden, am zweiten Knochenfragment (2) festlegbaren Knochenplatte (3), in deren Gewindebohrung (5) die Traghülse (6) mittels eines Außengewindes (9) einschraubbar ist, **dadurch gekennzeichnet, dass** das Knochengewinde (15) einerseits und die Gewindebohrung (5) und das Außengewinde (9) andererseits dieselbe Steigung der Gewindegänge (16; 17, 18, 19) aufweisen und dass die Gewindebohrung (5) sowie das Außengewinde (9) mindestens zwei nebeneinanderliegende Gewindegänge (17, 18, 19) mit entsprechend geringerer Breite jedes Gewindeganges (17, 18, 19) aufweisen.

2. Fixiersystem nach Anspruch 1, **dadurch gekennzeichnet, daß** die Gewindebohrung (5) und das Außengewinde (9) drei nebeneinanderliegende Gewindegänge (17, 18, 19) aufweisen.

3. Fixiersystem nach einem der voranstehenden Ansprüche, **dadurch gekennzeichnet, daß** die Knochenplatte (3) mehrere Gewindebohrungen (5) zur Aufnahme von Traghülsen (6) aufweist.

4. Fixiersystem nach einem der voranstehenden Ansprüche, **dadurch gekennzeichnet, daß** die Traghülse (6) eine kreiszylindrische Innenwand (10) aufweist, die sich eng an die kreiszylindrische Außenwand eines Schaftabschnittes (13) der Tragschraube (7) anlegt.

5. Fixiersystem nach Anspruch 4, **dadurch gekennzeichnet, daß** die kreiszylindrische Innenwand (10) und der kreiszylindrische Schaftabschnitt (13) sich zum Außengewindeende der Traghülse (6) hin stufenförmig erweitern, so daß die entstehende Stufe (11) einen Anschlag ausbildet, der die Ausziehbewegung der Tragschraube (7) aus der Traghülse (6) in Richtung auf das Knochengewinde (15) begrenzt.

6. Fixiersystem nach einem der voranstehenden Ansprüche, **dadurch gekennzeichnet, daß** die Tragschraube (7) an ihrem dem Knochengewinde (15) gegenüberliegenden Ende eine Aufnahmeöffnung (26) zum formschlüssigen Einführen eines Drehwerkzeuges (25) aufweist.

7. Fixiersystem nach einem der voranstehenden Ansprüche, **dadurch gekennzeichnet, daß** die Traghülse (6) an ihrem Außengewindeende eine Erweiterung (12) in ihrer Innenwand (10) aufweist zum formschlüssigen Einführen eines Drehwerkzeuges (27).

## Claims

1. Orthopedic fixation system for securing a first bone fragment (1) to a second bone fragment (2) with a support screw (7) adapted to be screwed into the first bone fragment (1) by means of a bone thread (15) having a thread turn (16), with a support sleeve (6) surrounding the support screw (7) and guiding this so as to be freely displaceable in longitudinal direction and with a bone plate (3) having at least one threaded bore (5) and being securable to the second bone fragment (2), the support sleeve (6) being adapted to be screwed into the threaded bore (5) of said bone plate by means of an external thread (9), **characterized in that** the bone thread (15), on the one hand, and the threaded bore (5) and the external thread (9), on the other hand, have the same pitch of the thread turns (16; 17, 18, 19), and that the threaded bore (5) as well as the external thread (9) have at least two thread turns (17, 18, 19) located next to one another with a correspondingly smaller width of each thread turn (17, 18, 19).

2. Fixation system as defined in claim 1, **characterized in that** the threaded bore (5) and the external thread (9) have three thread turns (17, 18, 19) located next to one another.

3. Fixation system as defined in either one of the preceding claims, **characterized in that** the bone plate (3) has several threaded bores (5) for receiving support sleeves (6).

4. Fixation system as defined in any one of the preceding claims, **characterized in that** the support sleeve (6) has a circular cylindrical inner wall (10) abutting closely on the circular cylindrical outer wall of a shaft section (13) of the support screw (7).

5. Fixation system as defined in claim 4, **characterized in that** the circular cylindrical inner wall (10) and the circular cylindrical shaft section (13) widen in a step-like manner towards the external thread end of the support sleeve (6) so that the resulting step (11) forms a stop limiting the withdrawal movement of the support screw (7) out of the support sleeve (6) in the direction towards the bone thread (15).

6. Fixation system as defined in any one of the preceding claims, **characterized in that** the support screw (7) has a receiving opening (26) for the form locking introduction of a rotary tool (25) at its end located opposite the bone thread (15).

7. Fixation system as defined in any one of the preceding claims, **characterized in that** at its external thread end the support sleeve (6) has an enlargement (12) in its inner wall (10) for the form locking introduction of a rotary tool (27).

## Revendications

1. Système de fixation orthopédique pour l'immobilisation d'un premier fragment d'os (1) sur un deuxième fragment d'os (2), comprenant une vis porteuse (7) pouvant être vissée dans le premier fragment d'os (1) au moyen d'un filetage pour os (15), qui présente un filet de filetage (16), le système comprenant par ailleurs un fourreau porteur (6), qui entoure la vis porteuse (7) et guide celle-ci de manière librement coulissante dans la direction longitudinale, et comprenant finalement une plaque d'ostéosynthèse (3), qui présente au moins un alésage fileté (5) et peut être immobilisée sur le deuxième fragment d'os (2), et dans l'alésage fileté (5) de laquelle peut être vissé le fourreau porteur (6) au moyen d'un filetage extérieur (9),
**caractérisé en ce que** le filetage pour os (15) d'une part et l'alésage fileté (5) et le filetage extérieur (9) d'autre part, présentent le même pas des filets de filetage (16 ; 17, 18, 19), et **en ce que** l'alésage fileté (5) ainsi que le filetage extérieur (9) présentent au moins deux filets de filetage (17, 18, 19) placés côte à côte avec une largeur moindre adéquate de chaque filet de filetage (17, 18, 19).

2. Système de fixation selon la revendication 1, **caractérisé en ce que** l'alésage fileté (5) et le filetage extérieur (9) présentent trois filets de filetage (17, 18, 19) placés côte à côte.

3. Système de fixation selon l'une des revendications précédentes, **caractérisé en ce que** la plaque d'ostéosynthèse (3) présente plusieurs alésages filetés (5) destinés à recevoir des fourreaux porteurs (6).

4. Système de fixation selon l'une des revendications précédentes, **caractérisé en ce que** le fourreau porteur (6) présente une paroi intérieure (10) cylindrique circulaire, qui s'applique étroitement contre la paroi extérieure cylindrique circulaire d'un tronçon de corps allongé (13) de la vis porteuse (7).

5. Système de fixation selon la revendication 4, **caractérisé en ce que** la paroi intérieure (10) cylindrique circulaire et le tronçon de corps allongé (13) cylindrique circulaire s'élargissent, de manière étagée en gradin, en direction de l'extrémité à filetage extérieur du fourreau porteur (6), de sorte que le gradin (11) ainsi formé réalise une butée, qui limite le mouvement d'extraction de la vis porteuse (7) hors du fourreau porteur (6), en direction du filetage pour os (15).

6. Système de fixation selon l'une des revendications précédentes, **caractérisé en ce que** la vis porteuse (7) présente, à son extrémité opposée à celle où se trouve le filetage pour os (15), une ouverture d'accueil (26) pour l'insertion, par complémentarité de formes, d'un outil de rotation (25).

7. Système de fixation selon l'une des revendications précédentes, **caractérisé en ce que** le fourreau porteur (6) présente, à son extrémité à filetage extérieur, un élargissement (12) dans sa paroi intérieure (10), pour l'insertion, par complémentarité de formes, d'un outil de rotation (27).
